Europäisches Patentamt

European Patent Office

Office européen des brevets

(11) Publication number: **0 255 367**
**A2**

# EUROPEAN PATENT APPLICATION

(21) Application number: **87306716.9**

(22) Date of filing: **29.07.87**

(51) Int. Cl.⁴: **C 12 P 21/00**
**C 12 N 15/00, G 01 N 33/577**

(30) Priority: **29.07.86 JP 179757/86**

(43) Date of publication of application:
**03.02.88 Bulletin 88/05**

(84) Designated Contracting States:
**AT BE CH DE ES FR GB GR IT LI LU NL SE**

(71) Applicant: **Yamashina, Ikuo**
**2 Shimogamohigashimorigamae-cho Sakyo-ku**
**Kyoto-shi Kyoto (JP)**

(72) Inventor: **Yamashina, Ikuo**
**2 Shimogamohigashimorigamae-cho Sakyo-ku**
**Kyoto-shi Kyoto (JP)**

(74) Representative: **Bizley, Richard Edward et al**
**BOULT, WADE & TENNANT 27 Furnival Street**
**London EC4A 1PQ (GB)**

(54) Cancer cell specific monoclonal antibodies and corresponding hybridomas, and their preparation and use.

(57) The invention provides a monoclonal antibody recognising human intestinal cancer cells, e.g. MLS 102 derived from a hubridoma obtainable from ECACC 86070307 specifically recognising glycoprotein sugar chain of such cells, more specifically NeuAcα2→6GalNAc. Hybridomas producing such antibodies may, for example, be prepared by fusing splenic cells of a mouse immunized by human intestinal cancer cells with myeloma cells.

EP 0 255 367 A2

**Description**

# CANCER CELL SPECIFIC MONOCLONAL ANTIBODIES AND CORRESPONDING HYBRIDOMAS, AND THEIR PREPARATION AND USE

This invention relates to intestinal cancer cell specific monoclonal antibodies, preferably to a monoclonal antibody designated MLS 102 specifically recognising a cancer-associated sugar chain antigen and to a hybridoma producing it. More specifically, this invention pertains to a monoclonal antibody MLS 102 which binds specifically to cancer cells derived from human intestinal cancer tissue, in which MLS 102 binds to a sialic acid-containing sugar chain of a glycoprotein but not to that of a glycolipid; MLS 102 also binds to a sugar chain NeuAc 2 6GalNAc which is abundant in ovine or bovine submaxillary mucin. This invention also pertains to a hybridoma MLS 102 which grows stably in the abdominal cavity of mice and produces the monoclonal antibody MLS 102.

It has been recognised that on the surface of cells there are complex carbohydrates such as glycoproteins, glycolipids and proteoglycans, whose sugar chains are altered as normal cells are transformed into cancer cells. Elucidation of the mutation, that is, the change of sugar chain structure has been attempted mainly by chemically analysing the sugar chain. However, it has become theoretically possible to obtain a monoclonal antibody which binds specifically to a changed sugar chain on the surface of a cancer cell since 1975 when Köhler and Milstein established the method of making a hybridoma and obtaining a monoclonal antibody therefrom. Recently, it has been reported that many monoclonal antibodies recognising a cancer-associated antigen recognise a sugar chain and, therefore, the significance of a monoclonal antibody recognising a cancer-associated sugar chain antigen has been pointed out and, further, some sugar chain structures of cancer-associated antigens have been determined. It is believed that such monoclonal antibodies may play a significant role in the clinical field such as in diagnosis, inspection and the therapy of cancer as well as in studies on cancer. Indeed, CA19-9, which is one of the cancer-associated sugar chain antigens can be assayed in the sera of cancer patients by using a monoclonal antibody NS19-9 specifically recognising CA19-9 (J. Magnani et al., J. Biol. Chem. 257, 14365 (1982)), which has been effectively utilised in diagnosis of pancreatic cancer.

Most conventional monoclonal antibodies provided hitherto bound to glycolipid on the surface of cancer cells since it is hard to prepare a monoclonal antibody binding to the sugar chain of a glycoprotein. Moreover, the structures of sugar chains recognised by antibodies have been determined by using glycolipid. However, it has been revealed that most cancer-associated sugar chain antigens appearing in the sera of cancer patients are expressed on the sugar chains of mucin-type glycoproteins because glycolipid is a minor component in sera. Therefore, a monoclonal antibody recognising the sugar chain of a glycoprotein has been desired as a cancer diagnostic for use in normal methods employing sera.

This invention provides a monoclonal antibody recognising a human intestinal cancer cell.

Monoclonal antibody MLS 102, an embodiment of this invention, was produced by a hybridoma which was selected as producing a monoclonal antibody binding to mucin-type glycopeptide prepared from LS180 (human intestinal cancer cell line, ATCC CL-187) from many hybridomas provided by fusing immunised mouse splenic cells with myeloma cells. Monoclonal antibodies, e.g. MLS 102, of this invention recognise human intestinal cancer cells and, specifically, glycoprotein sugar chain, which seemed hard to achieve in the prior art. More specifically, MLS 102 recognises NeuAc 2 6GalNAc, which is abundant in human intestinal cancer tissues and ovine and bovine submaxillary mucins.

Monoclonal antibody MLS 102 of this invention binds specifically to cancer cells but not to normal cells in human intestinal cancer tissue. Further, it binds to ovarian, gastric and esophageal cancer cells and may also bind to other human cancer cells.

The monoclonal antibody of the present invention does not bind to glycolipid, that is, it does not have any specificity for a crude glycolipid fraction extracted from bovine or porcine brain, LS180 cell, SW1116 cell (ATCC CCK233) or human meconium. It is well known that SW1116 cell is a cell line of human intestinal cancer and produces CA19-9 (J. Magnani et al., the same as mentioned above). Taking the above-mentioned properties of this antibody into consideration, it can be said that the antibody of this invention has a specific affinity to glycoprotein sugar chain. Most conventional monoclonal antibodies recognising cancer-associated sugar chain antigens have an affinity to sugar chain glycolipid. Therefore, the antibody of this invention can be regarded as a novel type of monoclonal antibody having new properties. Moreover, this antibody does not bind to glycolipid from SW1116 and so it is concluded that it is different from NS19-9.

The monoclonal antibody of this invention also has an affinity to glycoprotein in human meconium. It is well known that some cancer-associated antigens are temporarily expressed in infancy and, therefore, they are called carcinoembryonic antigens. In this respect, the relationship between carcinogenesis and infancy has been noted. Since the antibody of this invention binds to glycoprotein in the meconium, it is concluded that the sugar chain antigen recognised by this antibody is expressed both in human intestinal cancer tissue and in human infant intestinal tissue.

The monoclonal antibody of this invention also has an affinity to mucin type glycoproteins prepared from ovine, bovine or porcine submaxillary glands. Up to this time only two kinds of antigens, Forssman antigen and H-D antigen, were known as antigens which were expressed in other species of animal as well as in a human cancer. However, antigens expressed in the submaxillary mucin of other species of animal such as the antigen

0 255 367

recognised by the antibodies of this invention, have not been known up until this invention.

Furtheremore, monoclonal antibodies of this invention can have an affinity to a sugar chain of the structure NeuAc 2 6GalNAc. The higher the density of the sugar chain of this structure becomes, the more the affinity of the antibody increases. The sugar chain of the structure NeuAc 2 6GalNAc is observed in normal human intestinal tissue in such a small quantity that the antibody has a low affinity to normal human intestinal tissue. In contrast, the above chain of two sugars is frequently expressed and abundant in human intestinal cancer tissue, and, therefore, this antibody binds only to cancer tissue. These two sugars are the main constituents of ovine and bovine submaxillary mucin and ovine submaxillary mucin especially contains them in a higher density. This accords with the fact that the monoclonal antibody of this invention binds to ovine, bovine and porcine submaxillary mucins (in order of decreasing affinity).

A hybridoma producing a monoclonal antibody of this invention may be prepared by fusing myeloma cells with splenic cells (prepared from mice immunised by peritoneally inoculating human intestinal cancer cell LS180 thereinto according to the usual method) and selecting a clone which has an affinity to mucin-type glycopeptide isolated and purified from LS180 cells. The hybridoma can be stably stored in 10% dimethylsulfoxide and 90% fetal calf serum (FCS) in liquid nitrogen (-190°C) for a long time. As occasion demands, a part of the hybridoma may be thawed and incubated in RPMI-1640 or Dulbecco's MEM containing 20% FCS for its growth, and then the resultant may be inoculated into the abdominal cavity of a mouse to which pristane (0.5ml/mouse) has been administered 2 weeks to 3 months earlier to give a large amount of a monoclonal antibody of this invention in the ascites 10 to 14 days after inoculation of the hybridoma. In this method 7 to 14mg of the monoclonal antibody can be stably prepared from about 5ml of ascites per mouse. This monoclonal antibody in the ascites can be readily purified by applying it to an affinity column such as protein A-sepharose 4B after fractionation with ammonium sulfate and subsequent dialysis. Even when incubating the hybridoma in a culture medium, monoclonal antibody MLS 102 is secreted into the medium in a concentration of 20 to 40μg/ml about 3 days after incubation and, therefore, it can also be recovered from the medium.

Hybridoma MLS 102 has been deposited with European Collection of Animal Cell Cultures (ECACC) under accession number 86070307 under the Budapest Treaty since July 3, 1986.

Thus it will be appreciated that the invention provides a hybridoma capable of secreting a monoclonal antibody as defined earlier, e.g. MLS 102. The invention further provides a process for producing a hybridoma capable of secreting a monoclonal antibody which recognises a human intestinal cancer cell comprising fusing myeloma cells and antibody-secreting cells which have been sensitised by human intestinal cancer cells and selecting for fusions which can generate antibody having affinity for human intestinal cancer cells.

Also included in the invention is a method for producing a monoclonal antibody which recognises a human intestinal cancer cell which comprises growing a hybridoma as above such that it secretes monoclonal antibody, e.g. antibody MLS 102 (from hybridoma MLS 102 which is obtainable from ECACC 86070307).

The antibodies of the invention may be used in the provision of reagents or, in a manner known per se in the diagnosis or monitoring of cancer. The invention thus includes the use of an antibody in the provision of an analytical or diagnostic reagent or in the diagnosis or monitoring of cancer using samples taken from a patient.

In the accompanying drawings:-

FIG. 1 shows the affinity of G-50I and ovine glycoprotein, bovine and porcine submaxillary mucins (described as OSM, BSM and PSM) and meconium to MLS 102 bound to polystyrene beads. The ordinate gives the amount of bound $^{125}$I-MLS 102. The abscissa indicates the amount of a sample added as an amount of sialic acid.

FIG. 2 shows an activity of OSM, BSM, PSM and G-50I in inhibiting the binding of MLS 102 to G-50I. The ordinate gives the amount of bound $^{125}$I-protein A. The abscissa indicates the amount of mucin added as an amount of sialic acid.

FIG. 3 shows the activity of OSM and sugar chain prepared from OSM by hydrazine decomposition in inhibiting the binding of MLS 102 to G-50I. The ordinate and the abscissa have the same significance as in Fig. 1.

FIG. 4 shows the relation between the number of a fraction and [$^{14}$C] glucosamine radioactivity (dpm x $10^{-3}/20$μl).

The following Examples detail but do not restrict this invention.

EXAMPLE 1 - Preparation of Hybridoma

LS180 cell and BAlb/c mouse are used as antigen source and as the mouse to be immunised. Into a Balb/c mouse 1 to 2 x $10^6$ LS180 cells, which were incubated in Eagle medium containing 10% FCS, suspended in 0.5 ml of physiological saline are peritoneally administered 9 times in 3 months. Next, 1 to 2 x $10^6$ LS180 cells in 0.5ml of physiological saline are intravenously administered into the mouse to obtain splenic cells from the mouse 3 days after the administration. The splenic cells (1.5 x $10^8$) and myeloma cells (2 x $10^7$, sp2/0-Ag14) are mixed in Dulbecco's medium without FCS and centrifuged at 1,000 r.p.m. for 5 min for washing. A solution (0.5 ml) containing 35% polyethyleneglycol (PEG) #1000 is mixed with the Dulbecco's medium with FCS. The resulting mixture is centrifuged at 1,000 r.p.m. for ca. 5 min to fuse the splenic cells with the myeloma cells. The PEG used for the fusing is diluted with 5ml of Dulbecco's medium without FCS and 5ml of Dulbecco's medium with 20% FCS and removed by centrifuging at 1,000 r.p.m. for 5 min. The cells without PEG are suspended in 50ml of HT medium comprising 72% Dulbecco's medium and 8% NCTC 109 medium, 1 drop (c.a. 0.1ml) of which is placed in each well of a 96 well tissue culture plate and incubated for 24 hrs. Then, 1 drop of HT medium containing 0.8 M aminopterine is added to each well to change the medium to HAT medium. Hybridomas prepared by the fusing are selected from unfused splenic cells and myeloma cells by incubation in HAT medium. About 10 days after fusing the supernatant of each well is collected and assayed if it has an affinity to G-50I (see Referential example) adsorbed on a well according to the method shown in Example 4. The clones showing an affinity to the G-50I are further subjected twice to cloning by the dilution method to give several kinds of hybridomas. Monoclonal antibodies produced by these hybridomas were assayed by affinity to human intestinal cancer tissue. One antibody prepared this way, namely MSL 102, showed specific affinity to the cancer cells.

EXAMPLE 2 - Preparation and Purification of Monoclonal antibody

To a Balb/c mouse is peritoneally administered 0.5ml of pristane. The hybridoma, e.g. that which produces MLS 102, is incubated in a hybridoma growth medium comprising 10% Dulbecco's medium, 10% NCTC 109 medium and 20% FCS, $10^7$ cells of which are suspended in 0.5ml of Hanks' solution and peritoneally administered to the above mouse about 1 month after the administration of pristane. About 10 days later the ascites are recovered from the mouse. Each mouse has about 5 to 7ml of ascites containing about 10mg of MLS 102. Ammonium sulfate is added to these ascites to bring it to 50% saturation. The mixture is centrifuged and the precipitate is dissolved in 0.1M borate buffer solution containing 0.14M sodium chloride (pH 8.2, Buffer A) and dialysed against the same buffer. The resultant is divided into several portions, whch are applied to a protein A-Sepharose CL-4B column and washed with Buffer A. The column is previously equilibrated with Buffer A and contains 6ml of a resin. This column can absorb about 30mg of IgG. The antibody bound to the column is eluted with 0.1M acetic acid containing 0.15M sodium chloride. The eluate is neturalised with 1M Tris. To the eluate is added ammonium sulfate to bring it to 50% saturation. After centrifugation the precipitate is dissolved in Buffer A and dialysed against Buffer A to give a monoclonal antibody, e.g. MLS 102.

EXAMPLE 3 - Affinity of Monoclonal Antibody MSL 102 to Human Intestinal cancer tissue.

Human intestinal cancer tissue fixed with formalin is dehydrated and fixed with paraffin. Thin slices are prepared from the tissue and the paraffin is removed with xylene from the slice. The xylene is washed out with alcohol. Finally, the slice is washed with phosphate buffered saline (PBS) and soaked in PBS. Monoclonal antibody MLS 102 is allowed to bind to the slice overnight. The slice is washed and allowed to react with anti-mouse IgG antibody labelled with Fluorescein isothiocyanate (FITC). After washing out unbound FITC-IgG, the slice is observed under a fluorescence microscope. As a result, MLS 102 does not bind to normal tissue at all and specifically binds to mucin-like substance secreted by cancer cells. The result shows that MLS 102 recognises a cancer-associated antigen.

EXAMPLE 4 - Affinity of Monoclonal Antibody MLS 102 to Mucin-Type Glycopeptide.

To a poly(vinyl chloride)-well coated with mucin-type glycopeptide (G50-I) prepared from human intestinal cancer cell LS180 through polylysine and glutaraldehyde is added 1% bovine serum albumin and allowed to stand for 1 hr. After washing, monoclonal antibody MLS 102 is added to the well and allowed to react overnight. After washing the well, $^{125}$I-labelled protein A is added to the well and allowed to react for 2 hrs, followed by washing the well. Affinity of MLS 102 to G-50I is assayed by measuring the radioactivity of $^{125}$I bound to the well. As a result it was revealed that MLS 102 binds to G-50I. The same experiment was done by using G-50I from which sialic acid was removed and MLS 102 had no affinity to such G-50I. this means that MLS 102 binds to sugar chains containing sialic acid of mucin-type glycopeptides.

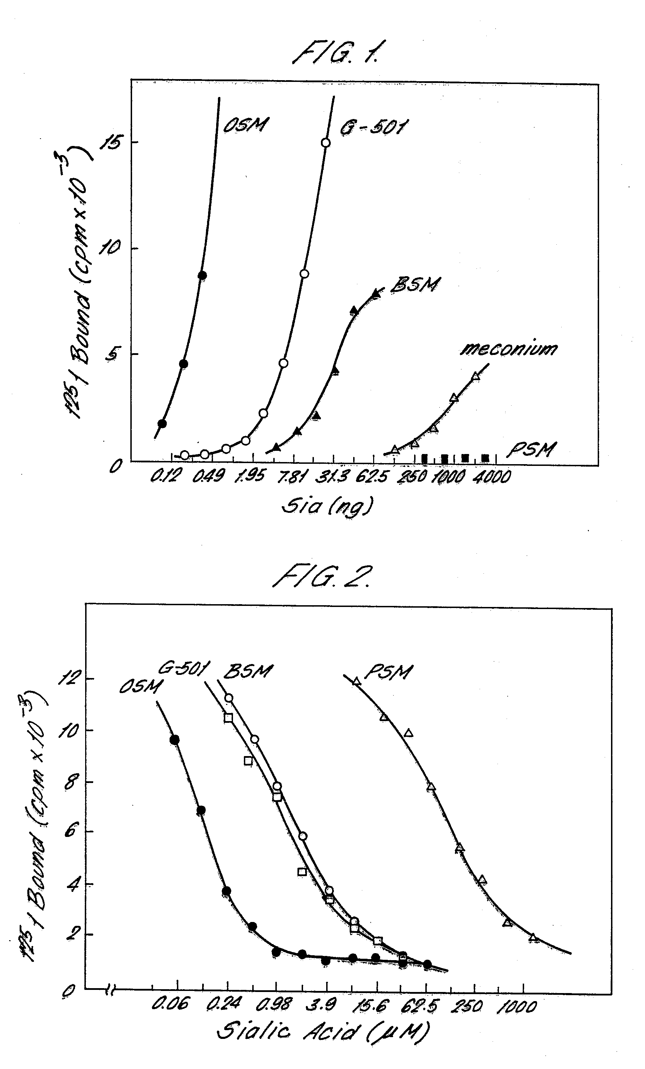

The affinity of MLS 102 may be assayed by using polystyrene beads immobilising MLS 102. Polystyrene beads (EP-03, #80, Sekisui Chemical Co., Ltd.) are washed with phosphate buffered saline (PBS, pH7.4) containing 0.15M sodium chloride. The beads are allowed to stand in PBS containing 0.1mg/ml of MLS 102 at 4°C overnight and coated with the antibody. The beads are washed 3 times with PBS containing 5% bovine serum albumin (BSA), soaked in the same solution and allowed to stand for 1 hr. After removing the solution by sucking, 100μl of sample solution (containing G-50I), 50μl of PBS containing 0.2% Tween 20 and 50μl of PBS containing $^{125}$I-MLS 102 (12.5ng) and 0.1% BSA (c.a. 200,000 counts) are well mixed with the beads and allowed to stand at 4°C overnight, followed by washing the beads 3 times with PBS. The radioactivity bound to the beads is measured by a γ-counter to calculate the affinity to the antibody (FIG. 1).

EXAMPLE 5 - Affinity of Monoclonal Antibody to Ovine, Bovine or Porcine Submaxillary Mucin.

Mucin prepared from ovine, bovine or porcine submaxillary gland is allowed to react with polystyrene beads coated with MLS 102 according to the method of Example 4 to assay the affinity of MLS 102 to those. As a result, it is revealed that MLS 102 binds to ovine, bovine and procine mucin, in order of decreasing affinity (FIG. 1 and Table 1).

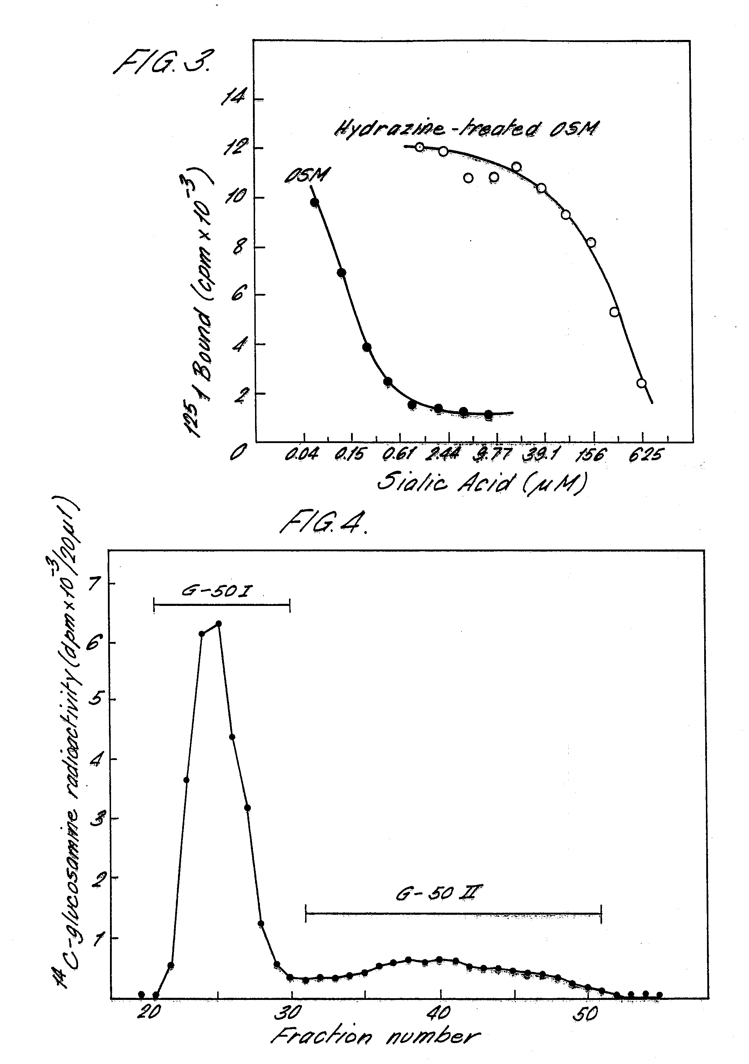

To each poly(vinyl chloride)-well coated with G-50I are added monoclonal antibody MLS 102 and submaxillary mucin of varying concentrations and allowed to stand overnight. According to the method of Example 4 the affinity of the antibody to G-50I is assayed by measuring the affinity of $^{125}$I-labelled protein A. Similar to the result of the above assay using beads, ovine, bovine and porcine submaxillary mucins inhibit the binding of the antibody to G-50I (in which order the activity decreases (FIG. 2)). This means that the antibody binds to ovine, bovine and porcine submaxillary mucins, in order of decreasing affinity.

EXAMPLE 6 - Affinity of Monoclonal Antibody MLS 102 to Glycoprotein in Meconium.

In 10ml of water is suspended 1.9mg of meconium and then 90ml of a mixture of chloroform/methanol (2:1, V/V) is added thereto, stirred well and allowed to stand to give an organic phase (under layer) and an aqueous phase (upper layer). The upper layer is dried and extracted 3 times with chloroform/methanol mixture (2:1, V/V). The extract and the under layer as noted above are combined to give a fraction of glycolipid in meconium, which is dried to be used in the following Example 7. To the residue of the extraction is added water and the mxiture is stirred and centrifuged to remove insoluble materials to give the supernatant as a glycoprotein fraction of the meconium. The affinity of MLS 102 to this glycopeptide fraction is assayed by using polystyrene beads as noted above and it is revealed that MLS 102 binds to this glycoprotein (FIG. 1).

EXAMPLE 7 - Affinity of Monoclonal Antibody MLS 102 to Colostrum, Semen, Cord Blood, Saliva and Amniotic Fluid.

Every sample as captioned was prepared from a normal human. The affinity of MLS 102 to each sample is assayed by the method using polystyrene beads as noted in Example 4. MLS 102 dis not bind to any samples (Table 1).

EXAMPLE 8 - Affinity of Monoclonal Antibody MLS 102 to Glycolipid of Bovine or Porcine Brain, LSS180 Cell and Meconium.

Bovine or porcine brain and LS180 cells are each homogenised in water at 4°C in a Dounce homogeniser. To the homogenate is added methanol and chloroform in such a way that the final ratio of chloroform:methanol:water becomes 2.7:5.4:2 (V/V). The mixture is stirred at room temperature for 30 min to extract glycolipids. After centrifugation, to the precipitate is added 2 volumes of water and the mixture is homogenised. Then, 8 volumes of a mixture of chloroform/methanol (1:2 V/V) is added thereto to extract glycolipid with stirring, followed by centrifuging to give the extract. After, the resulting two extracts are filtered through sellaite 535 and the filtrates are combined in a separatory funnel, to which water is added in such a way that the ratio of chloroform:methanol:water becomes 1:2:2.4 (V/V). After gently stirring and allowing to stand, the upper layer is removed and the under layer is well mixed with 3 volumes of methanol. Then, 2 volumes of 0.001M potassium chloride is added thereto, gently stirred and allowed to stand overnight to give an upper layer. To a mixture of this upper layer and the upper layer as mentioned above is added isobutanol and the mixture dried under reduced pressure. The dried preparation in a mixture of chloroform/methanol/water (60:30:4.5 V/V) is stirred overnight and centrifuged to remove insoluble materials and the resulting supernatant is dried. The dried preparation is dissolved in a small quantity of water and dialysed against water. After drying, the dried preparation is dissolved in methanol and centrifuged to remove insoluble materials to give a supernatant as a glycolipid fraction.

A glycolipid fraction of meconium was prepared as in Example 6. To each glycolipid fraction is added methanol in such a way that glycolipid in 1mg (wet weight) of starting material is dissolved in 20μl of methanol. To a poly(vinyl chloride)-well is added 20μl of the methanol solution. The methanol is evaporated under reduced pressure to coat the glycolipid on the surface of the well. To the well is added 1% bovine serum albumin and incubated for 30 mins. After washing the well, monoclonal antibody MSL 102 is added thereto and allowed to react at room temperature for 3 hrs. After washing the well, $^{125}$I-protein A is added thereto and allowed to react for 2 hrs. After washing the well, the affinity of MLS 102 is assayed by measuring the radioactivity bound to the well. MLS 102 did not bind to any glycolipid fractions (Table 1).

EXAMPLE 9 - Structure of Sugar Chain Recognised by Monoclonal Antibody MLS 102.

The structure of the sugar chain recognised by MLS 102 may be investigated by using ovine submaxillary mucin which has the highest affinity to MLS 102. A solution of a dried preparation of the mucin in anhydrous hydrazine is allowed to react at 100°C for 9 hrs. By this treatment the linkage between sugar and peptide in the mucin is cleaved and the sugar chain is isolated as reducing sugar. The sugar chain is gel-filtrated through sephadex G-25. The affinity of MLS 102 to each fraction is assayed by measuring the activity of each fraction in inhibiting the binding of MLS 102 to a well coated with bovine submaxillary mucin. The fraction containing main sugar chains was identical with the fraction having an affinity to MLS 102. When the sugar chain was further applied to several types of filter paper chromatography and high-voltage filter paper electrophoresis, the same results were observed.

An inhibition test was done by using the main glycopeptide of ovine submaxillary mucin, NeuAcα2→6Gal-NAcα→Ser, and its isomer, NeuAcα2→6GalNAcβ→Ser, both of which are artificially synthesised. As a result, both had the same affinity to the anitbody and, therefore, it was concluded that MLS 102 has an affinity to NeuAcα2→6GalNAc, which is a main sugar chain of ovine submaxillary mucin.

When the sugar chain of ovine submaxillary mucin is isolated therefrom by hydrazine degradation, inhibitory activity is dramatically reduced. It is inferred from this result that the affinity of MLS 102 deeply depends upon the density of the disaccharide NeuAcα2→6GalNAc on the polypeptide (FIG. 3).

## Table 1
## Affinity of MLS 102

| Sample | Affinity |
|---|---|
| G-50I | + |
| Ovine submaxillary mucin | + + |
| Bovine submaxillary mucin | + |
| Porcine submaxillary mucin | ± |
| Glycoprotein in meconium | + |
| Glycolipid of LS180 | − |
| Glycolipid of bovine brain | − |
| Glycolipid of porcine brain | − |
| Glycolipid of meconium | − |
| Colostrum | − |
| Semen | − |
| Cord blood | − |
| Saliva | − |
| Amniotic fluid | − |

REFERENCE EXAMPLE - Preparation of G-50I

G-50I is a glycopeptide derived from mucine-type glycoprotein produced by the human intestinal cancer cell line LS180 (ATCC CL-187), and may be prepared according to the following method.

Human intestinal cancer cell line LS180 (ATCC CL-187) is incubated in Dulbecco's medium containing 10% fetal calf serum for 7 days and recovered. The collected cells are repeatedly washed with phosphate buffered saline (PBS, 137mM sodium chloride, 2.7mM potassium chloride, 8.1mM disodium hydrogenphosphate, 1.5mM potassium dihydrogenphosphate, 1.0mM calcium chloride and 0.5mM magnesium chloride). Then, PBS containing 1% Triton X-100 (Rohm & Haas) is added thereto and the mixture is stirred under ice-cooling. After centrifugation, the supernatant is dialysed and freeze-dried. The resultant is defatted with chloroform/methanol (2:1) and suspended in acetate buffer containing 0.01M calcium acetate. Then, 1/50 parts of Pronase P (Kaken Pharmaceutical Co., Ltd.) to the above dried preparation is added thereto, followed by adding a small amount of toluene. The mixture is allowed to stand at 37°C for 3 days for sufficient digestion of protein. To the solution which becomes almost clear is added the same volume of 10% trichloroacetic acid. After stirring, the mixture is allowed to stand and centrifuged to remove insoluble materials. The supernatant is well mixed with the same volume of ether and centrifuged. The supernatant is removed and to the under layer (aqueous phase) further ether is added. The centrifugation is repeated 3 times. After it is confirmed that the aqueous phase is at pH 5, it is applied to a column (1.3 x 60cm) of sephadex G-25 pre-equilibrated with 0.5M pyridine-acetate buffer (pH 5.0) and developed with the same buffer. The eluate is collected by means of a fraction collector and the fractions positive in the orcinol-sulfuric acid reaction are combined and freeze-dried. The resulting dried powder is dissolved 0.5M pyridine-acetate buffer (pH 5.0), gel-filtrated by a column of sephadex G-50 and fractionated into 5ml portions by means of a fraction collector to give fractions (G-50I) passing through the G-50 column. These fractions usually contain glycopeptide derived from glycoprotein of mucin-type (0-glycoside type). FIG. 4 shows the relation between the number of a fraction and [14C] glucosamine radioactivity (dpm x $10^{-3}/20\mu l$).

The fractions 20-29 are combined and lyophilised to remove the solvent. The resulting powder is treated in the same manner as described above except for using sephadex G-200 to give practically pure mucin type glycopeptide G-50I.

As explained above, the present antibodies, e.g. monoclonal antibody MLS 102, enable us to make a precide decision in the diagnosis of various cancers, especially intestinal cancer. Further it becomes possible to provide the monoclonal antibody whenever it is necessary since the hybridoma producing the antibody can be preserved for a long period of time and can be proliferated stably in the abdominal cavity of a mouse. Therefore, the antibodies of this invention, e.g. monoclonal antibody MLS 102, and the hybridoma producing them bring an excellent effect to the diagnosis and therapy of cancer.

**Claims**

1. A monoclonal antibody recognising a human intestinal cancer cell.

2. A monoclonal antibody according to Claim 1 wherein said human intestinal cancer cell is LS180 (obtainable from ATCC CL-187).

3. A monoclonal antibody according to Claim 1 which recognises glycopeptide produced by said human intestinal cancer cell, e.g. G-50I.

4. A monoclonal antibody according to Claim 3 which recognises a sugar chain of said glycopeptide, e.g. NeuAcα2→6GalNAc.

5. A monoclonal antibody according to any one of claims 1 to 4 which recognises mucin-type glycoprotein prepared from ovine, bovine or porcine submaxillary gland or glycoprotein prepared from human meconium.

6. A monoclonal antibody according to any one of claim 1 to 5 which does not recognise glycolipid prepared from bovine or porcine brain, human intestinal cancer cell LS180 (obtainable from ATCC CL-187) or human meconium or normal human colostrum, semen, cord blood, saliva or amniotic fluid.

7. Monoclonal antibody MLS 102 which may be produced by hybridoma MLS 102 obtainable from ECACC 86070307).

8. A hybridoma capable of secreting an antibody according to any one of claims 1 to 6.

9. Hybridoma MLS 102 (obtainable from ECACC 86070307).

10. The use of an antibody according to any one of claims 1 to 7 in the provision of an analytical or diagnostic reagent or in the diagnosis or monitoring of cancer using samples taken from a patient.

Claims for the following contracting states: Austria, Greece and Spain

1. A process for producing a hybridoma capable of secreting a monoclonal antibody which recognises a human intestinal cancer cell comprising fusing myeloma cells and antibody-secreting cells which have been sensitised by human intestinal cancer cells and selecting for fusions which can generate antibody having affinity for human intestinal cancer cells.

2. A process according to claim 1 wherein the cells used for sensitisation are cells of LS180 (obtainable from ATCC CL-187).

3. A process according to claim 2 wherein the antibody-secreting cells are splenic cells.

4. A process according to any one of claims 1 to 3 wherein the antibody-secreting cells are derived from mice and sensitisation is achieved by peritoneally inoculating human intestinal cancer cells.

5. A process according to any one of claims 1 to 4 wherein selection of fusions is effected by screening for clones having an affinity for glycopeptide produced by human intestinal cancer cells, e.g. G-50I.

6. A process according to claim 5 wherein a selected clone has an affinity for a sugar chain of said glycopeptide, e.g. NeuAc$\alpha$2→6GalNAc.

7. A process according to claim 5 or claim 6 where a selected clone recognises mucin-type glycoprotein prepared from ovine, bovine or porcine submaxillary gland or glycoprotein prepared from human meconium and/or does not recognise glycolipid prepared from bovine or porcine brain, human intestinal cancer cell LS180 (obtainable from ATCC CL-187) or human meconium or normal human colostrum, semen, cord blood, saliva or amniotic fluid.

8. A process according to claim 1 wherein the hybridoma resulting from the selection has the characteristics of MLS 102 (obtainable from ECACC 86070307).

9. A method for producing a monoclonal antibody which recognises a human intestinal cancer cell which comprises growing a hybridoma which has been produced by a process according to any one of claims 1 to 8 such that it secretes monoclonal antibody, e.g. antibody MLS 102 (from bybridoma MLS 102 which is obtainable from ECACC 86070307).

10. The use of an antibody which recognises a human intestinal cancer cell, or an antibody which has been produced by a process according to claim 9, in the provision of an analytical or diagnostic reagent or in the diagnosis or monitoring of cancer using sample(s) taken from a patient.

0255367

FIG. 1.

FIG. 2.

0255367

FIG.3.

Hydrazine-treated OSM

OSM

$^{125}I$ Bound (cpm × $10^{-3}$)

Sialic Acid (μM)

FIG.4.

$^{14}C$-glucosamine radioactivity (dpm × $10^{-3}$/20μl)

G-50 I

G-50 II

Fraction number